# EUROPEAN PATENT APPLICATION

(11) **EP 2 182 381 A1**
(43) Date of publication of application: **05.05.2010**
(21) Application number: 09173218.0
(22) Date of filing: 16.10.2009
(51) Int. Cl.: G01S 7/52, G01S 15/89, A61B 5/00

(54) **Ultrasound image display with additional information using PPG and ECG signals**

(30) Priority: 31.10.2008 KR 20080107476
(71) Applicant: Medison Co., Ltd., Kangwon-do 250-870 (KR)
(72) Inventor: Park, Joong Hoon, 135-851 Seoul (KR)
(74) Representative: Schmid, Wolfgang

(57) **Abstract**

An embodiment for displaying an ultrasound image with additional information using a photoplenthysmography (PPG) signal and an electrocardiogram (ECG) signal in an ultrasound system is disclosed. The ultrasound system includes a photoplenthysmography (PPG) signal generating unit and an electrocardiogram (ECG) signal generating unit. The PPF signal generating unit and the ECG signal generating unit generate a PPG signal for the target object and an ECG signal for the target object, respectively. An additional information forming unit forms additional information by using the PPG signal and the ECG signal. A display unit displays ultrasound images of the target object together with the additional information.

## Description

The present application claims priority from Korean Patent Application No. 10-2008-0107476 filed on October 31, 2008, the entire subject matter of which is incorporated herein by reference.

### TECHNICAL FIELD

The present disclosure generally relates to ultrasound systems, and more particularly to an ultrasound image display with additional information using a photoplenthysmography (PPG) signal and an electrocardiogram (ECG) signal in an ultrasound system.

### BACKGROUND

Photoplethysmography (PPG) is known as an optical measurement technique that can obtain physiologic information from a target object such as blood oxygen saturation (SpO2), heart rate, etc. PPG is often performed by using a pulse oximeter. The pulse oximeter is commonly worn on a relative narrow portion of a human body (e.g., finger, ear, etc.) and emits an infrared light and a red light thereto. The pulse oximeter may compute a ratio of red to infrared light absorption of hemoglobin and oxyhemoglobin, and measures variance of blood volume by using the computed ratio to thereby obtain the physiologic information. The physiologic information is important in examining the status of a patient.

Generally, additional information such as physiologic information and a PPG waveform is very useful for examining a patient. Unfortunately, the conventional ultrasound system does not provide such additional information. Thus, there has been a need for an ultrasound system that can provide such additional information to perform more accurate examination.

### SUMMARY

An embodiment for ultrasound image display with additional information using a photoplenthysmography (PPG) signal and an electrocardiogram (ECG) signal is disclosed herein. In one embodiment, by way of non-limiting example, an ultrasound system comprises: a transmission/reception (Tx/Rx) unit operable to transmit ultrasound signals to a target object and receive echo signals reflected from the target object to thereby output receive signals; an ultrasound image forming unit operable to form ultrasound images based on the receive signals; a photoplenthysmography (PPG) signal generating unit operable to generate a PPG signal for the target object; an electrocardiogram (ECG) signal generating unit operable to generate an ECG signal for the target object; an additional information forming unit operable to form additional information by using the PPG signal and the ECG signal; and a display unit to display the ultrasound images and the additional information.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

- FIG. 1: is a block diagram showing an illustrative embodiment of an ultrasound system.
- FIG. 2: is a schematic diagram showing an example of displaying ultrasound images and additional information.
- FIG. 3: is a schematic diagram showing an example of computing pulse transit times by using a PPG waveform and an ECG waveform.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

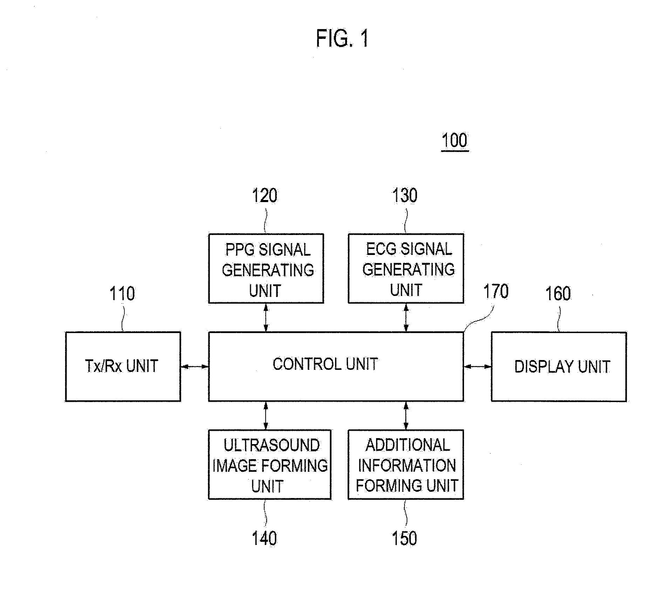

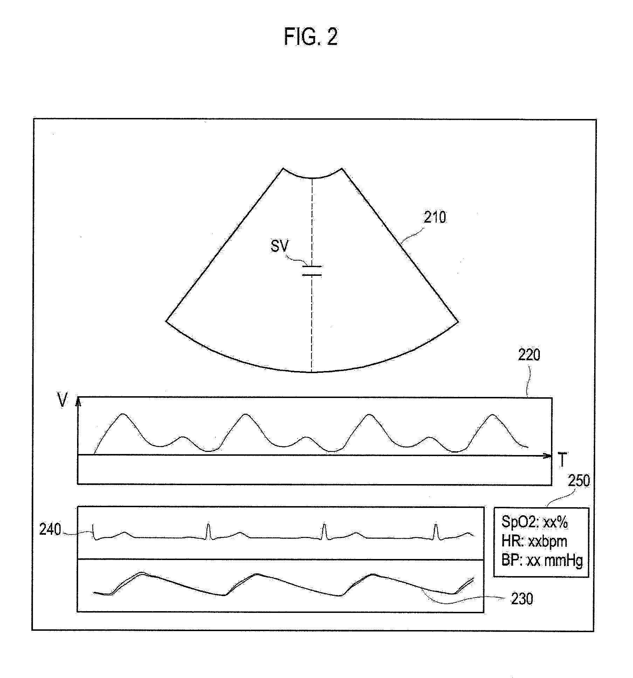

FIG. 1 is a block diagram showing an illustrative embodiment of an ultrasound system 100. As shown in FIG. 1, the ultrasound system 100 may include a transmission and reception (Tx/Rx) unit 110. The Tx/Rx unit 110 may be operable to transmit ultrasound signals to a target object and receive echo signals reflected from the target object. The Tx/Rx unit 110 may be further operable to form receive signals based on the echo signals. In one embodiment, the receive signals may include first receive signals obtained in a B-mode and second receive signals obtained in a D-mode. The second receive signals may be obtained by repeatedly transmitting the ultrasound signals to a region of interest, e.g., a sample volume SV (*see* FIG. 2).

In one embodiment, the Tx/Rx unit 110 may include a transmit signal generator (not shown) that may be operable to generate a plurality of Tx signals for transmission of the ultrasound signals. The Tx/Rx unit 110 may further include an ultrasound probe (not shown). The ultrasound probe may include an array transducer consisting of a plurality of elements. The array transducer may be either 1D or 2D array transducer, although it is certainly not limited thereto. The array transducer may be operable to generate ultrasound signals in response to the Tx signals. Also, the array transducer may be further operable to convert the echo signals into electrical receive signals.

The Tx/Rx unit 110 may further include a beam former (not shown) that is coupled to the ultrasound probe. The beam former may be operable to perform analog-to-digital conversion upon the electrical receive signals to thereby output digital receive signals. The beam former may be also operable to apply delays to the digital receive signals in consideration of positions of the transducer elements and focal points, and then may sum the delayed digital receive signals to thereby output a plurality of receive-focused beams.

The ultrasound system 100 may further include a photoplethysmography (PPG) signal generating unit 120. The PPG signal generating unit 120 may include at least one PPG sensor (not shown). The PPG sensor may be worn on a specific portion of the target object such as a finger, toe, ear, etc. The PPG sensor may emit an infrared light and a red light to the specific portion to thereby detect a blood flow therefrom. The PPG sensor may be operable to generate the PPG signal based on the blood flow detection. The PPG sensor may include a pulse oximeter and the like.

The ultrasound system 100 may further include an electrocardiogram (ECG) signal generating unit 130. The ECG signal generating unit 130 may be operable to generate an ECG signal. In generating the ECG signal, any electrocardiographic device, which is well known to a person skilled in the art, may be used. Thus, detailed descriptions thereof will be omitted herein.

The ultrasound system 100 may further include an ultrasound image forming unit 140. The ultrasound image forming unit 140 may be operable to form an ultrasound image based on the receive signals, which are provided from the Tx/Rx unit 110. In one embodiment, the ultrasound system 100 may be operable to form a B-mode image 210 based on the first receive signals and a Doppler-mode image 220 based on the second receive signals, as shown in FIG. 2.

The ultrasound system 100 may further include an additional information forming unit 150. The additional information forming unit 150 may be operable to form additional information such as physiologic information based on the PPG signal and the ECG signal. In one embodiment, the physiologic information may include blood oxygen saturation, heart rate and blood pressure. Referring to FIG. 2, the additional information forming unit 150 may be operable to form a PPG waveform 230 based on the PPG signal. Also, the additional information forming unit 150 may be operable to form an ECG waveform 240 based on the ECG signal. The additional information forming unit 150 may be further operable to form the physiologic information 250 including information on the blood oxygen saturation (SpO2), heart rate (HR) and blood pressure (BP) based on the PPG waveform 230 and the ECG form 240. The HR information may be obtained by using the characteristic of the PPG waveform that it is synchronized with a heart beat.

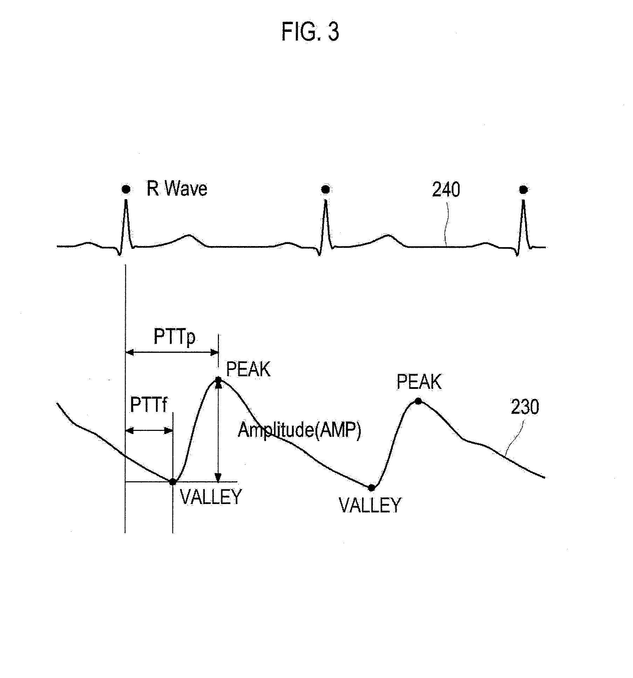

Referring to FIG. 3, the additional information forming unit 150 may be operable to detect peak points and valley points on the PPG waveform 230, as well as peak points on the ECG waveform 240. The additional information forming unit 150 may be further operable to compute an interval between the peak point of the PPG waveform 230 and the peak point of the ECG waveform 240 (hereinafter, referred to as first pulse transit time PPTₚ). The additional information forming unit 150 may be further operable to compute an interval between the valley point of the PPG waveform 230 and the peak point of the ECG waveform 240 (hereinafter, referred to as second pulse transit time PPT_{f}). In one embodiment, the additional information forming unit 150 may be operable to form the BP information by using the characteristic of the blood pressure that is inverse proportional to the computed first and second pulse transit times PPT_{P} and PPT_{f}. That is, if the blood pressure increases, then the distensibility of the blood wall decreases so that the first and second pulse transit times PPT_{P} and PPT_{f} may be reduced. On the other hand, if the blood pressure decreases, then the distensibility of the blood wall increases so that the first and second pulse transit times PPT_{P} and PPT_{f} may increase.

In one embodiment, the additional information forming unit 150 may include a PPG waveform forming section (not shown), an ECG waveform forming section (not shown) and a physiologic information forming section (not shown). The PPG waveform forming section may be operable to form the PPG waveform and the ECG waveform forming section may be operable to form the ECG waveform. The physiologic information forming section may be operable to form the physiologic information based on the PPG waveform and the ECG waveform.

Referring back to FIG. 1, the ultrasound system 100 may further include a display unit 160. The display unit 160 may be any display capable of displaying images such as an ultrasound image. The display unit 160 may be one of CRT, LCD, flat panel display and the like, although it is not limited thereto. The ultrasound images formed in the ultrasound image forming unit 140, i.e., the B-mode image 210 and the Doppler-mode image 220, may be displayed on a screen of the display unit 160, as shown in FIG. 2. Also, the additional information formed in the additional information forming unit 150, i.e., the PPG waveform 230, ECG waveform 240 and physiologic information 250, are displayed on the screen of the display unit 160.

The ultrasound system 100 may further include a control unit 170 that may be operable to control the transmission and the reception of the ultrasound signals. The control unit 170 may be further operable to control the formation and display of the ultrasound images. Also, the control unit 170 may be operable to control the generation of the PPG and ECG signals and the formation of the additional information. In one embodiment, the control unit 170 may be operable to control the display unit 160 such that the additional information such as the blood oxygen saturation, heart rate and blood pressure are displayed together with the ultrasound image on the screen of the display unit 160. Thus, the examination upon the heart using the ultrasound system may be more accurately performed.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
a transmission/reception (Tx/Rx) unit operable to transmit ultrasound signals to a target object and receive echo signals reflected from the target object to thereby output receive signals;
an ultrasound image forming unit operable to form ultrasound images based on the receive signals;
a photoplenthysmography (PPG) signal generating unit operable to generate a PPG signal for the target object;
an electrocardiogram (ECG) signal generating unit operable to generate an ECG signal for the target object;
an additional information forming unit operable to form additional information by using the PPG signal and the ECG signal; and
a display unit for displaying the ultrasound images and the additional information.

2. The ultrasound system of Claim 1, wherein the additional information include a PPG waveform, an ECG waveform and physiologic information.

3. The ultrasound system of Claim 2, wherein the physiologic information include blood oxygen saturation, heart rate and blood pressure.

4. The ultrasound system of Claim 3, wherein the additional information forming unit includes:
a PPG waveform forming section operable to form the PPG waveform by using the PPG signal;
an ECG waveform forming section operable to form the ECG waveform by using the ECG signal; and
a physiologic information forming section operable to form the physiologic information by using the PPG waveform and the ECG waveform.

5. The ultrasound system of Claim 4, wherein the physiologic information forming section is configured to:
detect peak points and valley points on the PPG waveform and peak points on the ECG waveform;
compute a first pulse transit time representing an interval between the peak point of the PPG waveform and the peak point of the ECG waveform and a second pulse transit time representing an interval between the valley point of the PPG waveform and the peak point of the ECG waveform; and
form information on the blood pressure by using the first and second pulse transit times.
